# EUROPÄISCHE PATENTSCHRIFT

(11) **EP 0 214 975 B1**
(45) Veröffentlichungstag und Bekanntmachung des Hinweises auf die Patenterteilung: **10.03.1993**
(21) Anmeldenummer: 85902454.9
(22) Anmeldetag: 09.05.1985
(51) Int. Cl.: G01N 33/60, G01N 33/94, C07C 405/00

(54) **VERFAHREN ZUR BESTIMMUNG GERINGER STOFFMENGEN VON ARZNEIMITTELN, VON KÖRPEREIGENEN ODER ANDEREN CHEMISCHEN SUBSTANZEN IN BIOLOGISCHEM MATERIAL**
PROCESS FOR THE DETERMINATION OF SMALL QUANTITIES OF DRUGS, BODY SUBSTANCES OR OTHER CHEMICAL SUBSTANCES IN A BIOLOGICAL MATERIAL
PROCEDE DE DETERMINATION DE PETITES QUANTITES DE MEDICAMENTS, DE SUBSTANCES SOMATIQUES OU D'AUTRES SUBSTANCES CHIMIQUES DANS UN MATERIAU BIOLOGIQUE

(30) Priorität: 10.05.1984 DE 3417638
(43) Veröffentlichungstag der Anmeldung: 25.03.1987
(73) Patentinhaber: SCHERING AKTIENGESELLSCHAFT, 13342 Berlin (DE)
(72) Erfinder: HÜMPEL, Michael, D-1000 Berlin 31 (DE); KRAUSE, Werner, D-1000 Berlin 27 (DE); SCHULZE, Paul-Eberhard, D-1000 Berlin 39 (DE); NIEUWEBOER, Bob, D-1000 Berlin 27 (DE)
(86) Internationale Anmeldenummer: DE8500157
(87) Internationale Veröffentlichungsnummer: WO8505452

(56) Entgegenhaltungen:
- AU-C- 495 838
- FR-A- 2 415 303
- FR-A- 2 426 262
- CHEMICAL ABSTRACTS, Band 99, Nr. 17, 24. Oktober 1983, Columbus, Ohio, US; W.KRAUSE et al.: "Pharmacokinetics and biotransformation of the prostacyclin analog, ZK 36.374 I. Synthesis of a tritium marker and excretion of [3H]-ZK 36.374 in the rat", Seite 128, Zusammenfassung Nr. 134 414r

## Beschreibung

Seit Jahren wird der Radioimmunoassay (RIA) als eine empfindliche Methode zur Bestimmung kleinster Mengen körpereigener Substanzen oder von Arzneistoffen verwendet. Wegen der sehr empfindlichen physikalischen Nachweismöglichkeit der hierfür verwendeten Radioisotope, wie zum Beispiel ¹³¹Jod, ¹²⁵Jod, Tritium und andere, ist diese Methode bisher unübertroffen. Nachteile zeigt dieser Test immer dann, wenn bei der Bestimmung von körpereigenen oder medikamentös zugeführten Fremdstoffen Stoffwechselprodukte infolge von Kreuzreaktionen mitbestimmt werden und so eine Aussage über den tatsächlichen Gehalt der zu bestimmenden Stoffe erschwert oder unmöglich gemacht wird. Diese Kreuzreaktionen sind prinzipiell nie auszuschließen. Sie sind letztlich abhängig von der Selektivität des verwendeten Antiserums. In vielen Fällen sind die im Plasma entstehenden Metaboliten zugeführter Arzneimittel unbekannt; demzufolge ist eine Testung des Antikörperserums auf Spezifität nicht möglich.

Neben dieser Unsicherheit in der Spezifität bringt auch noch die Verwendung von radioaktivem Material Probleme in der Handhabung der RIA-Methode mit sich. Es müssen behördlich genehmigte und für eine gefahrlose Handhabung der radioaktiven Substanzen geeignete Räumlichkeiten vorhanden sein. Man ist deshalb seit längerem bemüht, eine Bestimmungsmethode zu finden, die ohne radioaktiven Tracer auskommt, die frei ist von einer möglichen Kreuzreaktion und bei der die Spezifität des RIA's noch erhöht wird.

In J. of Chromatography 223 (1981) 193 wird die Verwendung eines an Sepharose kovalent gebundenen 17ß-Östradiol-Antiserums zur Extraktion von 17ß-Ostradiol aus biologischem Material beschrieben. Für die Bestimmung wird jedoch noch mit Tritium-markierten Verbindungen gearbeitet.

L. Siekmann et al., Z. Anal. Chem. **252**, 294 (1970) beschreiben die quantitative Bestimmung geringer Steroidkonzentrationen in Körpertlüssigkeiten unter Zusatz der entsprechenden deuterierten Substanz zu der undeuterierten zu bestimmenden Substanz mit Hilfe der GC-MS-Methode und anschließender Ether-Chloroform-Exraktion.

Es wurde nun gefunden, daß man kleinste Stoffmengen von Arzneistoffen oder von körpereigenen Substanzen auch ohne die entsprechenden radioaktiven Tracer und bei gleichbleibender Spezifität des Antikörpers ermitteln kann, wenn man den Antikörper an eine stationäre Phase chemisch koppelt und ein deuteriertes Analogon der nachzuweisenden Verbindung verwendet. Die Bestimmung erfolgt mit Hilfe einer mit Massenspektometrie gekoppelten Gaschromatographie. Die Spezifität dieser neuen Bestimmungsmethode ist gegenüber der bekannten RIA-Methode deutlich erhöht.

Während beim RIA die nachzuweisende Verbindung mit dem radioaktiven Tracer bei der in vitro-Bestimmung um den Antikörper konkurriert und somit hoch spezifisch zugesetzt werden muß, wird bei dem neuen Verfahren der deuterierte Tracer der biologischen Probe zugesetzt und über eine geeignete stationäre Phase, die den Antikörper im Überschuß enthält, quantitativ abgetrennt. Hierbei werden sowohl die im biologischen Material befindliche, zu messende nicht deuterierte Verbindung als auch der deuterierte Tracer quantitativ vom Antikörper festgehalten. Es ist für die nachfolgende Bestimmung unerheblich, ob der Antikörper auch noch andere Stoffe zu binden vermag (Kreuzreaktion). Für die sich anschließende Messung ist nur das Verhältnis zu bestimmende nicht deuterierte Substanz/deuterierte Verbindung von Bedeutung. Nachdem das Plasma die stationäre Phase (mit dem Antikörper) passiert hat, wird mehrmals mit einer geeigneten Waschflüssigkeit (in den meisten Fällen mit einem der Phase zugrundeliegenden Lösungsmittel mit den in der Biochemie üblichen Zusätzen von NaHCO₃, Essigsäure und dem Fachmann geläufigen Puffern), wie zum Beispiel isotonische Kochsalzlösung oder Wasser, nachgewaschen. Anschließend werden die deuterierte Verbindung und die nicht deuterierte Verbindung von der stationären Phase entfernt und das Verhältnis der beiden Stoffe mit einem Massenspektrometer bestimmt.

Das erfindungsgemäße Verfahren hat gegenüber den bekannten Verfahren den Vorteil, daß
1.) die Bestimmung ohne Radioaktivität durchgeführt werden kann,
2.) evtl. Kreuzreaktionen des Antikörpers mit Drittstoffen (Metaboliten) unerheblich sind,
3.) seine Spezifität die des RIA's übersteigt und
4.) der gekoppelte Antikörper mehrmals verwendet werden kann. Bei 50 Extraktionen waren keinerlei Zersetzungen zu erkennen.
Als stationäre Phase verwendet man vorzugsweise polymere Trägermaterialien, wie zum Beispiel Sepharose, Cellulose, Carbopol, Silicagel usw. Als Trägermaterial ist jedoch auch Al₂0₃ (· ₓ H₂O) geeignet. Die Mengen an Antikörper, mit denen üblicherweise gearbeitet wird, betragen 50 µg bis 1 mg. Ein weiterer Vorteil des neuen Verfahrens ist seine Wirtschaftlichkeit. Während das bekannte RIA-Verfahren mit Radioisotopen arbeitet, deren Einsatz wegen der besonderen Schutzmaßnahmen stets mit einem im unvergleichlich hohen Aufwand verbunden ist, können die für das neue Verfahren erforderlichen deuterierten Verbindungen ohne besondere Schutzmaßnahmen hergestellt werden, und ihr Einsatz in dem neuen Verfahren ist deshalb völlig problemlos. Die Messung im Massenspektrometer gehört inzwischen zur täglichen Praxis eines gut geschulten Laborpersonals und bedarf deshalb keiner weiteren Erläuterung, da hierbei lediglich die wiedergefundene, nicht deuterierte Verbindung in unmittelbare Relation zur zu bestimmenden Verbindung gesetzt wird.

Das neue Verfahren eignet sich für alle Verbindungen, für die ein Antikörper vorhanden ist, und zwar sowohl für körpereigene Substanzen, wie natürliche Prostaglandine oder Steroidhormone, als auch für körperfremde Verbindungen, wie z. B. Arzneimittel oder chemische Schadstoffe.

Als Tracer können nicht nur deuterierte Verbindungen, sondern auch die Substanzen eingesetzt werden, die mit stabilen Isotopen, wie ¹³C, ¹⁵N, ¹⁸O oder ähnlichen Isotopen markiert werden können.

Als stationäres Trägermaterial kann jede polymere Matrix eingesetzt werden, an die der Antikörper gekoppelt werden kann.

Anstelle von GC/MS kann auch jedes andere Verfahren angewendet werden, mit dem das Verhältnis nicht deuterierte Verbindung/deuterierte Verbindung bestimmt werden kann. Dazu gehören z. B. MS allein oder MS-MS.

Sollte keine mit stabilen Isotopen markierte Substanz zur Verfügung stehen, so kann auch eine andere, chemisch ähnliche Verbindung verwendet werden. Voraussetzung ist jedoch, daß sie mit dem Antikörper kreuzreagiert. In diesem Fall muß sich an die Extraktion mit dem gekoppelten Antikörper ein weiteres Trennverfahren, wie z. B. GC oder HPLC anschließen, bevor die beiden Substanzen bestimmt werden können.

Sollte weder eine isotopenmarkierte noch eine kreuzreagierende Verbindung zur Verfügung stehen, so kann auch eine andere, chemisch ähnliche Substanz eingesetzt werden, die dann erst nach der Antikörper-vermittelten Extraktion dem Extrakt zugesetzt wird. Im Falle von Iloprost wurde dies mit Carbacyclin überprüft.

Zur GC/MS-Bestimmung wurden dann die beiden Massen m/z 493 und m/z 503 gemessen. Alle übrigen Bedingungen sind unverändert. Der Variationskoeffizient ändert sich in diesem Fall geringfügig auf 2,6 % (n = 5) bei 50 pg-Proben.

Die Erfindung betrifft somit ein Verfahren zur Bestimmung geringer Stoffmengen von Arzneimitteln, von körpereigenen oder anderen chemischen Verbindungen in biologischem Material, das dadurch gekennzeichnet ist, daß man entweder die zu bestimmende Verbindung und ihr deuteriertes Analogon an einem im Überschuß vorliegenden Antikörper, der an eine stationäre Phase aus polymerem Material gekoppelt ist, bindet, und daß man die beiden Verbindungen vom Antikörper ablöst und das Verhältnis deuterierte Verbindung/nicht deuterierte Verbindung bzw. kreuzreagierende Verbindung massenspektometrisch mißt oder für den Fall, daß eine geeignete deuterierte oder kreuzreagierende Verbindung nicht zur Verfügung stehen sollte, als internen Standard auch eine chemisch ähnliche Verbindung verwendet, die jedoch nach der Antikörpervermittelten Extraktion dem extrakt hinzugefügt wird und für den Fall daß eine kreuzreagierend Verbindung oder eine chemisch ähnliche Verbindung verwendet wird man vor der Massenspektrometrie ein Trennverfahren einschaltet.

### Beispiel 1

Sythese von deuteriertem Iloprost-D₅ 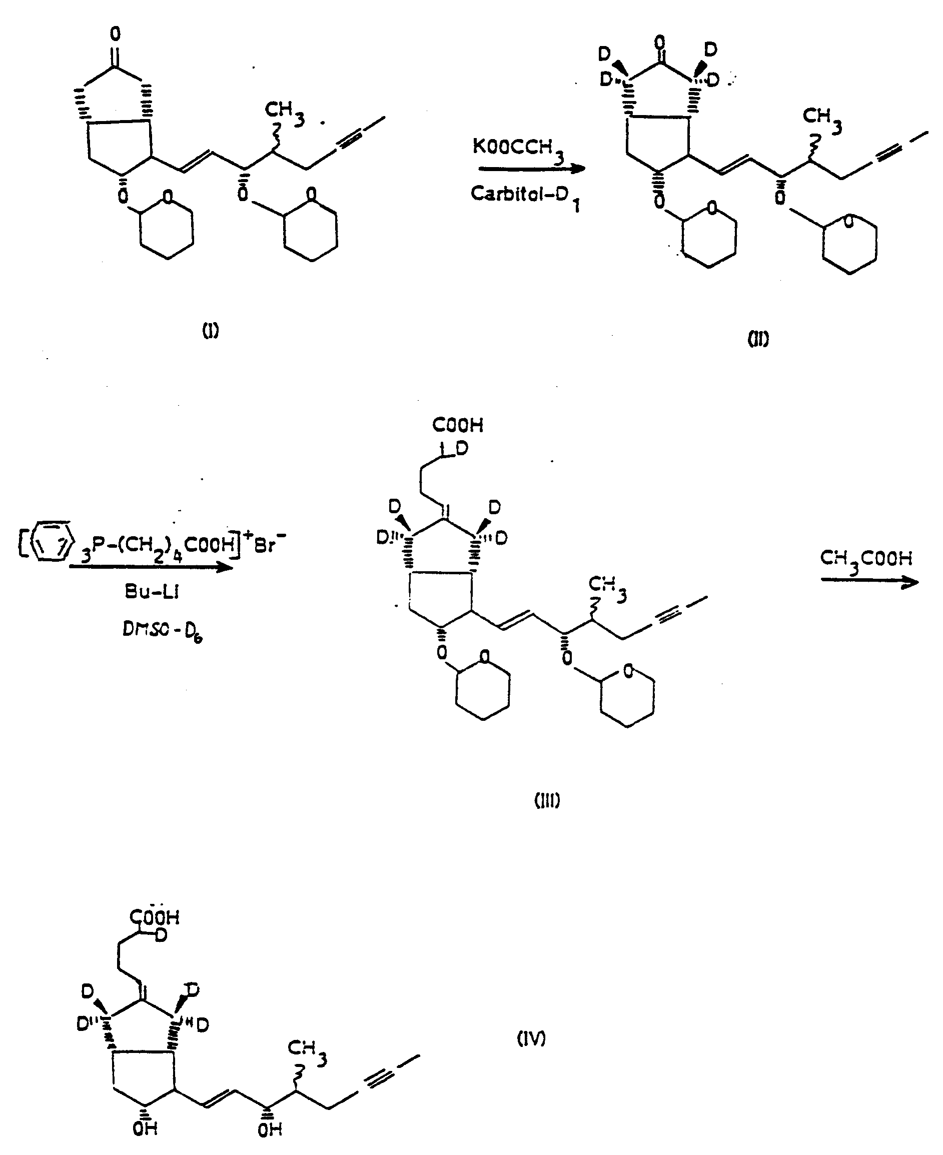
1 g (0.024 mMol) der Verbindung I wurde zu einer Lösung von 4.87 g (50 mMol) wasserfreiem Acetat in 130 ml (1.37 Mol) Carbitol-D₁ unter Durchleiten von trockenem Stickstoff gegeben. Es wurde bei Zimmertemperatur so lange gerührt, bis die Substanz gelöst war. Anschließend blieb die Lösung verschlossen und vor Licht geschützt 21 Tage bei Zimmertemperatur stehen. Zur Aufarbeitung wurden 46 ml entnommen, mit 30 ml D₂O versetzt und 3mal mit je 50 ml trockenem Hexan extrahiert. Die Hexan-Phasen wurden vereinigt, noch 2 mal mit je 50 ml

D₂O gewaschen, über Natriumsulfat getrocknet und eingeengt. Die Rohausbeute betrug 300 mg.

Die erhaltene Verbindung II zeigte im Massenspektrum folgende Deuterium-Verteilung:

| | |
|---|---|
| D₀ | 3.3 % |
| D₁ | 3.6 % |
| D₂ | 11.2 % |
| D₃ | 22.6 % |
| D₄ | 52.6 % |
| D₅ | 6.6 % |

4.41 g (9.95 mMol) 4-Carboxy-butyl-triphenylphosphonium-bromid wurden über Stickstoff in 9.12 Dimethylsulfoxid-O₆ und 4.62 ml Al₂O₃ getrocknetes Tetrahydrofuran gelöst. Dazu wurden bei 0 - 8 °C 2.2 g (19.9 mMol) Kalium-tert-butylat unter Rühren hinzugegeben.

Nach 30 min Rühren wurden 4.62 ml dieser Lösung zu der in 1 ml Tetrahydrofuran (über Al₂O₃ getrocknet) gelösten Verbindung II gegeben und unter Stickstoffbegasung noch 2 h bei 30 °C und anschließend über Nacht bei Zimmertemperatur gerührt. Der Reaktionsablauf wurde mittels Dünnschichtchromatographie im System Essigester/Hexan (3:2) überprüft.

Die Lösung wurde dann mit 25 ml 30%iger Zitronensäure und 50 ml Wasser versetzt und mit Methylenchlorid erschöpfend extrahiert. Nach Trocknen mit Natriumsulfat wurde eingeengt und der ölige Rückstand über eine Niederdrucksäule an Kieselgel gereinigt. Als Elutionsmittel wurde Toluol/Essigester in einem Gradientensystem (100:0 bis 70:30) verwendet. Die Ausbeute an reiner cis-Verbindung (III) betrug 105 mg.

Verbindung III wurde in 2 ml Tetrahydrofuran gelöst, mit 3 ml Wasser und 3 ml Eisessig versetzt und 24 h bei Zimmertemperatur gerührt. Nach Einengen im Vakuum wurde der Rückstand in 2 ml Methylenchlorid/Isopropanol(9:1) gelöst und über eine Niederdrucksäule gereinigt (10 g Kieselgel, Gradient Methylenchlorid/Isopropanol, 9:1 bis 7:3 in 2 h. 80 ml/h). Die Ausbeute an 5-{(E)-(1S,5S,6R,7R)-7-Hydroxy-6-[(E)-(3S,4RS)-3-hydroxy-4-methyl-oct-1-en-6-. inyl]-[2-D,2-D, 4-D, 4-D]-bicyclo[3.3.0]-octan-3-yliden}-[2-D]-pentansäure (IV) betrug 20 mg.

| Analytik und Strukturbestätigung | | |
|---|---|---|
| Massenspetrum: | D₀ | 2.8 % |
| | D₁ | 2.7 % |
| | D₂ | 7.3 % |
| | D₃ | 14.1 % |
| | D₄ | 29.2 % |
| | D₅ | 30.5 % |
| | D₆ | 3.1 % |
| | D₇ | 3.6 % |
| | D₈ | 2.0 % |
| | D₉ | 2.7 % |
| | D₁₀ | 2.1 % |
| HPLC: | Lichrosorb RP-18, 5 µm, 250 x 4.6 mm Methanol/Wasser/Eisessig, 70:30:0.6, 1.5 ml/min UV-Detektion bei 205 nm | |
| DC: | Kieselgel Mechylenchlorid/Isopropanol (9:1), Essigester (100] | |

Co-Chromatographie mit bekanntem Vergleichsmaterial ergab völlige Identität.

### Beispiel 2

### Kopplung des Antikörpers an die stationäre Phase

22 mg des in Prostagland. Leukotr. Med.10, 289 (1983) beschriebenen Antikörpers für Iloprost wurden an 2,4 g CNBr-aktivierte Sepharose 4B (Pharmacia) gekoppelt. Dazu wurde die Sepharose zunächst alt 500 ml 10⁻³ M Salzsäure gewaschen. Anschließend wurde der Antikörper in 0,1 M NaHCO₃-Lösung (+ 0,5 M NaCl) dazugegeben. Nach 2stündigem Rotieren wurde mit 0,1 M Acetatpuffer (pH 4, unter Zusatz von 1 M NaCl) und danach mit 0,1 M Boratpuffer (pH 8, 1 M NaCl) gewaschen. Überschüssige aktive Gruppen wurden mit 1 M Ethanolamin desaktiviert. Anschließend wurde noch zweimal mit Acetatpuffer bzw. Boratpuffer gewaschen. Der an Sepharose gekoppelte Antikörper wurde in 25 ml 0,1 M Phosphatpuffer (pH 7) bei 4 °C unter Lichtausschluß) gelagert.

### Beispiel 3

### Extraktion von Iloprost aus biologischem Material

0.88 mg des Sepharose-gekoppalten Antikörpers (entsprechend 1 ml Phosphatpuffer) werden in eine mit einem Wattepfropfen gegen Aus laufen gesicherte Pasteur-Pipette gegeben und mit Wasser, Aceton und wiederum Wasser gewaschen. Anschließend wird die biologische Phase, z.ß. Plasma, mit 300 pg D₅-Iloprost versetzt, in die Pasteur-Pipette gegeben und mit 10 ml Wasser nachgewaschen. Das Probenvolumen ist ohne Bedeutung, wie für bis 20 ml Plasma gezeigt werden konnte. Nach dem Waschvorgang wird das Gel mit Luft trockengeblasen und deuteriertes und nicht-deuteriertes Iloprost mit 10 ml Aceton/Wasser (95:5, v/v) eluiert. Nach Durchlaufen der ersten 5 ml wird die Pasteur-Pipette für 1 h verschlossen. Die erhaltenen Extrakte werden vereinigt und im Vakuum zur Trackene eingeengt. Der Rückstand wird für die GC/MS-Analyse derivatisiert.

Die Extraktionausbeute bei Verwendung des stationären Antikörpers betrug >90 %. Sie war unabhängig von der Iloprost-Konzentration.

Extraktionsausbeute aus 1 ml Plasma bei Verwendung von Tritium-markiertem Iloprost (n = 5):

| Konzentration (pg/ml) | Extraktionsausbeute (%) |
|---|---|
| 10 | 91 ∓ 2 |
| 100 | 91 ∓ 2 |
| 1000 | 93 ∓ 1 |

### Beispiel 4

### Derivatisierung

Der Rückstand wird in 100 µl Acetonitril (mit 5 % Methanol) aufgenommen und mit 10 µl einer Lösung von Pentarluorbenzylbromide (30 % in Acetonitril, w/v) und 10 N-Ethylbisisopropylamin versetzt. Nach 40 min bei 40 °C wird die Lösung mit einem Stickstoffstrom zur Trockene eingeengt und mit 10 µl BSTFA versetzt. Nach 30 min bei 50 °C ist die Probe zur GC/MS-Analyse bereit.

### Beispiel 5

### GC/MS-Analyse

Zur Messung des Verhältnisses nicht-deuteriertes Iloprost/deuteriertes Iloprost wurde ein GC/MS-Gerät vom Typ Finnigan 4021 benutzt. Es enthielt die folgenden Bausteine: GC (Typ 9610)., MS (Typ 4000) mit PPNICI-Einheit, INCOS 2100-Datensystem. Die gaschromatographische Trennung wurde auf einer CP-Sil 5 CB - Quarzkapillarsäule (25 m ID 0.23 mm, Filmdichte 0.12 µm, Fa. Chrompack), die direkt in das Massenspektrometer geleitet wurde, durchgeführt. Das Trägergas war Helium mit einem Eingangsdruck von 24 PSI. Die Proben wurden splitlos bei 290 °C injiziert. Das Temperaturprogramm der Säule begann bei 150 °C (1 min) und erreichte 310 °C mit einer Heizrate von 20 °C/min. Das Massenspektrometer wurde bei einer Temperatur von 240 °C betrieben. Die Elektronenenergie betrug 70 eV und der Emissionsstrom 0.2 µA. Der Sekudärelektronenvervielfacher war auf 1.5 kV eingestellt, der Vorverstärker auf 10⁻⁸A/V. Iloprost und D₅-Ilprost wurden mit NC I (negative Ionen - chemische Ionisation) und Einzelionennachweis (MID) bei m/z 503 bzw. 508 bestimmt.

### Beispiel 6

### Auswertung

Die Auswertung der Meßdaten erfolgt durch Vergleich der Peakflächen-Verhältnisse Iloprost/D₅-Iloprost mit einer Eichgeraden. Die Eichgerade erhält man durch Extraktion von Proben bekannter Konzentration und Auftragen des Peakflächenverhältnisses Iloprost/D₅ -Iloprost gegen die Konzentration:

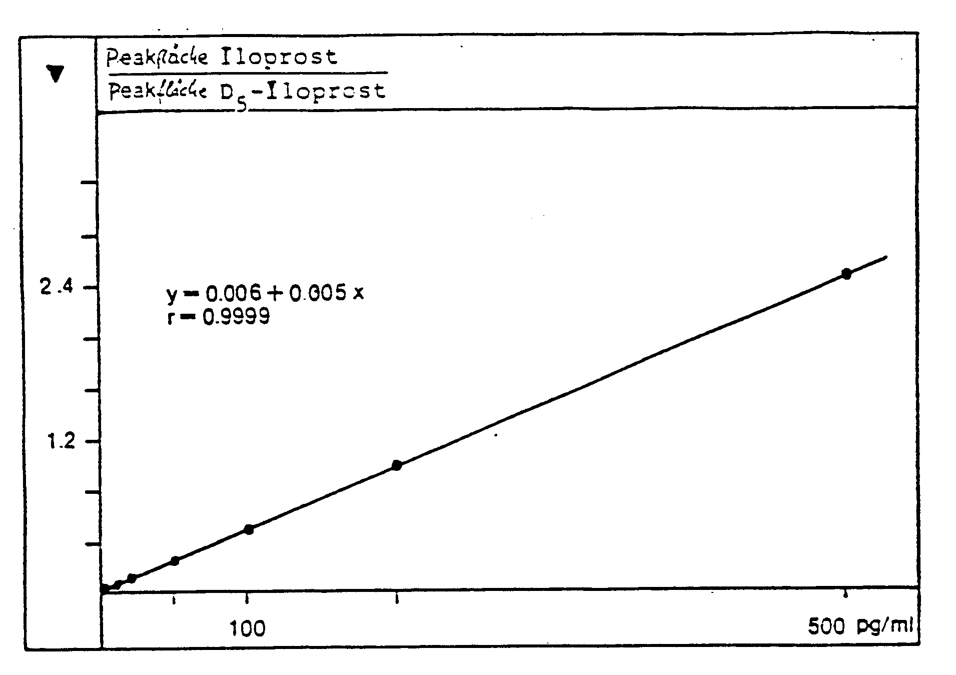

### Beispiel 7

Die höhere Spezifität des neuen Verfahrens im Vergleich zum Radioimmunoassay kann aus der nachstehenden Tabelle ersehen werden. Trotz Verwendung des gleichen Antikörpers waren die mit der neuen Methode gemessenen Konzentrationen deutlich niedriger als die mittels RIA bestimmten. Dies gilt vor allem für die späteren Zeitpunkte nach der Applikation, wo überwiegend (kreuzreagierende) Metaboliten im Plasma zu erwarten sind.

Vergleich der mittels RIA bzw. mittels des neuen Verfahrens gemessenen Iloporost-Konzentrationen bei 5 Versuchspersonen. Es wurden eine intravenöse Infusion von 1 oder von 3 ng/kg/min über 45 min oder eine orale Dosis von 1 µg/kg verabreicht. Die Plasmaproben aus jeweils einem frühen Zeitbereich (15 - 45 min bzw. 0 - 15 min) und einem späten Zeitbereich (60 - 90 min bzw. 50 - 90 min) wurden vereinigt und in diesen Mischproben die Iloprost-Konzentration gemessen.

| Probe | RIA | neues Verfahren |
|---|---|---|
| | (pg/ml) | |
| i.v. (1 ng/kg/min; 45 min) 15 - 45 min | 59 | 35 |
| i.v. (1 ng/kg/min; 45 min) 60 - 90 min | 24 | 4 |
| i.v. (3 ng/kg/min; 45 min) 15 - 45 min | 146 | 152 |
| i.v. (3 ng/kg /min; 45 min) 60-90 min | 49 | 13 |
| p.o. (1 µg/kg) 0 - 15 min | 166 | 123 |
| p.o. (1 µg/kg) 50-90 min | 99 | 20 |

### Beispiel 8

Die nachfolgende Abbildung illustriert die Plasmaspiegel von Iloprost, die mit Hilfe der Antikörper/GC/MS-Methode ermittelt wurden. Es werden die Iloprost-Plasmaspiegel von 5 männlichen Versuchspersonen dargestellt, die entweder eine 30minütige intravenöse Infusion von 1, 2 oder 8 ng/kg/min oder eine intragastrale Dosis von 1 oder 2,5 µg/kg verabreicht bekamen.

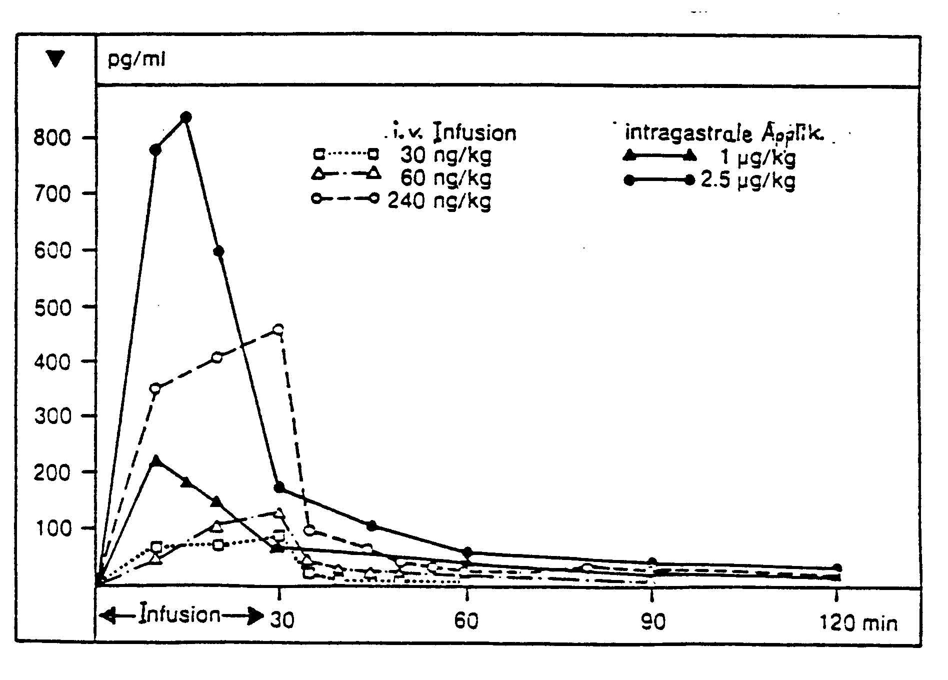

### Beispiel 9

Statt Iloprost-D₅ kann als interner Standard auch Carbacyclin verwendet werden. In diesem Fall muß der Standard nach der Extraktion zugefügt werden. Anstelle von m/z 508 wird m/z 493 im Massenspektrometer ermittelt. Die Reproduzierbarkeit differiert nur geringfügig von der mit deuteriertem Iloprost, wie aus dem nachstehenden Zahlenvergleich hervorgeht:

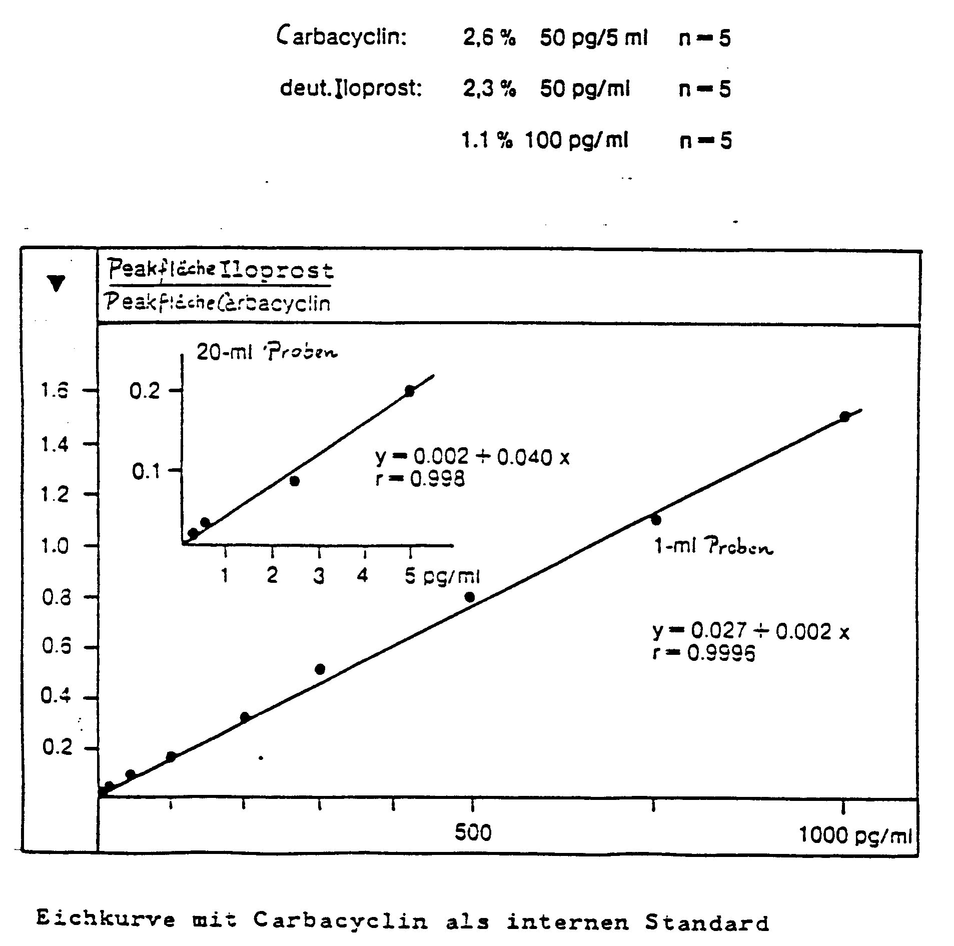

Der Variationskoeffizient betrug 2,3 % bei einer Konzentration von 50 pg/ml und 1,1 % bei 100 pg/ml (jeweils n = 5). Die Nachweisgrenze betrug ca. 5 pg/ml bei Verwendung von 1 ml Plasma und 0,25 pg/ml bei Einsatz von 20 ml Plasma. Die für den Radioimmunoassay mit dem gleichen Antikörper berechnete Nachweisgrenze war 20 pg/ml.

### Beispiel 10

Entsprechend den vorgenannten Beispielen wurde Östradiol bestimmt.

### Beispiel 11

Entsprechend den vorgenannten Beispielen wurde 11-Nor-9-tetrahydrocannabinol-9-carbonsäure (hauptsächlicher THC-Metabolit) bestimmt.

## Patentansprüche

1. Verfahren zur Bestimmung geringer Stoffmengen von Arzneimitteln, von körpereigenen oder anderen chemischen Verbindungen in biologischem Material, dadurch gekennzeichnet, daß man entweder die zu bestimmende Verbindung und ihr deuteriertes Analogon an einen im Überschuß vorliegenden Antikörper, der an eine stationäre Phase aus polymerem Material gekoppelt ist, bindet, und daß man die beiden Verbindungen vom Antikörper ablöst und das Verhältnis deuterierte Verbindung/nicht deuterierte Verbindung bzw. kreuzreagierende Verbindung massenspektometrisch mißt oder für den Fall, daß eine geeignete deuterierte oder kreuzreagierende Verbindung nicht zur Verfügung stehen sollte, als internen Standard auch eine chemisch ähnliche Verbindung verwendet, die jedoch nach der Antikörper vermittelten Extraktion dem Extrakt hinzugefügt wird und für den Fall daß eine kreuzreagierend Verbindung oder eine chemisch ähnliche Verbindung verwendet wird man vor der Massenspektrometrie ein Trennverfahren einschaltet.

## Claims

1. Process for the determination of small quantities of medicaments or of endogenous or other chemical compounds in biological material, characterized in that either the compound to be determined and its deuterated analog are bound to an antibody present in excess, which antibody is coupled to a stationary phase of a polymeric material, and the two compounds are separated from the antibody and the proportion of deuterated compound/non-deuterated compound or cross-reacting compound is measured by mass spectrometry; or, where a suitable deuterated or crossreacting compound is not available, a chemically similar compound is used as the internal standard, but is added to the extract after the antibody-mediated extraction, and, where a cross-reacting compound or a chemically similar compound is used, a separating process is inserted before the mass spectrometry.

## Revendications

1. Procédé destiné à doser de faibles quantités de médicaments, de composés endogènes ou d'autres composés chimiques dans un matériau biologique, caractérisé ou bien en ce qu'on fixe le composé à doser et son analogue deutérié à un anticorps présent en excès, lequel est couplé à une phase stationnaire constituée d'un matériau polymère, et en ce qu'on sépare les deux composés de l'anticorps et qu'on mesure par spectrométrie de masse le rapport entre le composé deutérié et le composé non-deutérié ou le compose subissant une réaction croisée ; ou bien en ce que, quand on ne dispose pas d'un composé approprié, deutérié ou pouvant subir une réaction croisée, on utilise aussi comme étalon interne un composé chimiquement analogue, qui cependant est ajouté à l'extrait après extraction médiée par l'anticorps, et, dans le cas où on utilise un composé pouvant subir une réaction croisée ou un composé analogue, on insère, avant la spectrométrie de masse, une opération de séparation.
